(19) **Europäisches Patentamt**

**European Patent Office**

**Office européen des brevets**

(11) **EP 1 426 096 A1**

(12) **EUROPEAN PATENT APPLICATION**

(43) Date of publication:
 **09.06.2004 Bulletin 2004/24**

(51) Int Cl.7: **B01D 53/78**, B01D 53/75,
 B01D 53/34, B01D 53/14,
 A61L 2/20

(21) Application number: **03027633.1**

(22) Date of filing: **02.12.2003**

| | |
|---|---|
| (84) Designated Contracting States:<br>**AT BE BG CH CY CZ DE DK EE ES FI FR GB GR HU IE IT LI LU MC NL PT RO SE SI SK TR**<br>Designated Extension States:<br>**AL LT LV MK**<br><br>(30) Priority: **03.12.2002 IT MI20022559**<br><br>(71) Applicant: **Air Futura S.r.l.**<br>**36016 Thiene (IT)** | (72) Inventors:<br>• **Bonfadini, Danilo Dante**<br>**26015 Soresina (IT)**<br>• **Bonfadini, Roberto**<br>**26015 Soresina (IT)**<br>• **Bonfadini, Elio Giacomo**<br>**26012 Castelleone (IT)**<br><br>(74) Representative: **Gervasi, Gemma, Dr.**<br>**Notarbartolo & Gervasi S.p.A.,**<br>**Corso di Porta Vittoria, 9**<br>**20122 Milano (IT)** |

(54) **Multiple effect apparatus and process for removing ethylene oxide from a gaseous mixture**

(57) This document describes a process for removing ethylene oxide from gaseous mixtures and an apparatus suitable for implementing this process. The removal process involves blowing the gaseous mixture, containing ethylene oxide, through a series of one or more bubblers (1), static filling columns (5), and nebulization columns (6); inside these items, the gaseous mixture comes into contact with a solution (3) able to decompose ethylene oxide. The gaseous mixture is bubbled and decontaminated thoroughly and quickly, removing ethylene oxide effectively.

## Description

## Technical field

This invention relates to sterilization performed with ethylene oxide. More particularly, it describes a process and an apparatus for removing ethylene oxide contained in gaseous mixtures used for decontaminating materials sterilized in ethylene oxide sterilizers.

## Background art

Ethylene oxide (epoxyethane dimethylene oxide) is a colourless gas obtained by oxidation of ethylene in the vapour phase. It contains a highly reactive peroxidic bond and is used as raw material to derive numerous, very useful products and intermediates including ethylene and polyethylene glycol, glycol ether, ethanolamine, ethylene cyanhydrin, hydroxyethyl cellulose and hydroxyethyl starch, polyols, polyethers, and so forth.

Ethylene oxide is highly effective in destroying bacteria, spores, fungus, and viruses. This gas works — the process is not yet understood completely — by penetrating into the microorganism and by alkylating the protein polar groups; this inactivates the proteins and blocks the enzymatic mechanisms vital for the life of the microorganism and its toxicity.

Ethylene oxide has been used for some time now as a biocidal gas for sterilizing food, fabric, packaging material, medical-surgical aids, and so forth. It is particularly useful for sterilizing dry-heat labile materials.

Sterilization methods for treating hospital products and medical devices have been implemented and perfected since 1940-50; today, ethylene oxide sterilizers are used extensively throughout the world.

Unfortunately, ethylene oxide is a flammable gas that is toxic to man and animals when not removed completely after sterilization; residual traces of this substance remain in or on the sterilized material. The symptoms of acute poisoning from this gas are pulmonary irritation, headaches, nausea, vomiting, dyspnoea, chest pain, drowsiness, and a loss of coordination. However, even when no acute symptoms are present, there are risks associated with chronic exposure to small quantities of the substance: the main toxicological risks are mutagenesis, teratogenesis, and carcinogenesis.

Thus, after the sterilization procedure is completed, the materials treated with ethylene oxide and the sterilization chambers must be aerated or ventilated with other gases to eliminate any traces of ethylene oxide; in turn, the air or gas used for ventilating must be treated to remove ethylene oxide before being released into the environment.

Different systems are used to reduce the levels of ethylene oxide contained in gaseous mixtures; some of these methods react ethylene oxide with acidic solutions to form ethylene glycol, a non-toxic product that can be disposed of easily.

However, the known methods, which involve reacting the gas with acidic solutions, do not offer very satisfactory results since they merely reduce ethylene oxide levels without completely eliminating all residues; furthermore, they involve a lengthy procedure, sometimes with several reducing cycles, leading to a significant waste of energy.

The increasing need to speed up the sterilisation cycle, and the growing demand to preserve the environment and the health and safety of workers and consumers render more and more urgent the need for improved systems to remove ethylene oxide from gaseous mixtures; this new procedure must eliminate, as quickly as possible, all traces of potentially harmful ethylene oxide.

## Objects of the invention

It is an object of this invention to provide a new process for removing ethylene oxide from gaseous mixtures, and an apparatus suitable for implementing this process. The removal process involves blowing the gaseous mixture containing ethylene oxide through a series of one or more bubblers, static filling columns, and nebulization columns; inside these items, the gaseous mixture comes into contact with a solution able to decompose ethylene oxide. The gaseous mixture is bubbled and decontaminated thoroughly and quickly, removing ethylene oxide effectively.

## Description of the drawings

Figure 1: diagram of the removal process
Figure 2: removal tower — side views: 2.1, 2.2, 2.3
Figure 3: removal tower — top view
Figure 4a: scrubber
Figure 4b: scrubber (detail view)

## Description of the invention

A first object of the present invention is a process for removing ethylene oxide contained in a gaseous mixture characterised in that said gaseous mixture is blown through one or more bubblers, one or more static filling columns, and one or more nebulization columns. Inside said items, the gaseous mixture comes into contact with a solution (hereinafter referred to as the "decomposing solution") able to convert ethylene oxide into non-toxic products.

The bubblers, the static filling columns, and the nebulization columns are connected to each other to form a circuit. The gaseous mixture containing ethylene oxide is blown through this circuit by means of a forced ventilation system. The gaseous mixture comes into contact with the contacting solution in a different manner in each part of the circuit, progressively reducing ethylene oxide levels; the gas coming out of the circuit is essentially free from ethylene oxide.

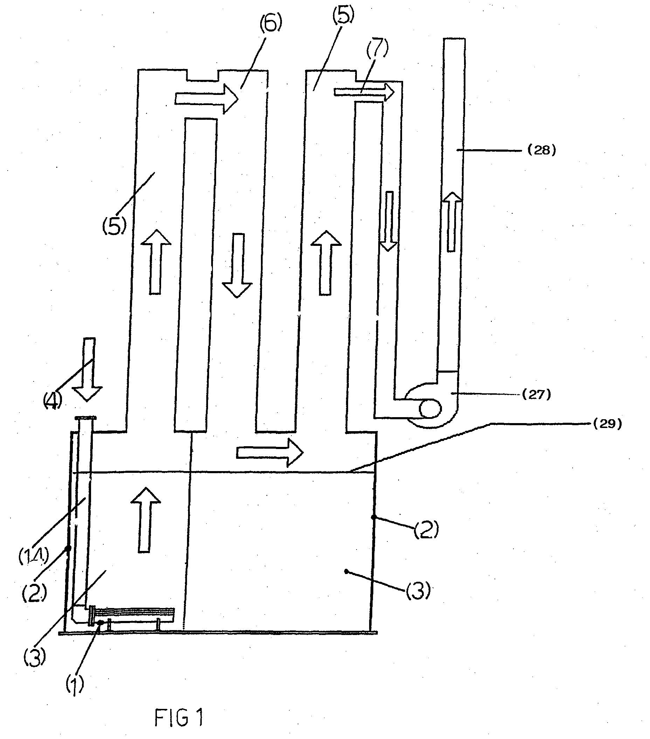

Figure 1 shows the removal process in accordance with the invention; this figure also shows a sketch of the apparatus used to implement the process.

The bubbler (1) is able to bubble the gaseous mixture through the decomposing solution (3) contained in a suitable tank (2). Preferably, the bubbler consists of a microperforated pipe placed in the tank and immersed in the decomposing solution; the gaseous mixture (4) containing ethylene oxide is forced to run through the bubbler; the gaseous mixture comes out of the holes and bubbles through the decomposing solution.

The static filling columns (5) are hollow, countercurrent columns: so-called because the decomposing solution flows against the flow of the gaseous mixture.

After entering the bottom of the column, the gas stream flows upward through the column. At the same time, the decomposing solution flows downward (i.e. in the opposite direction).

The nebulization columns (6) are hollow columns in which the decomposing solution is nebulized.

Nebulization is preferably carried out by nozzles fitted on different levels of the column: preferably, at the base, at mid height, and at the top. Thus, the gaseous mixture, flowing upward through the column, comes into contact with the nebulized decomposing solution.

The removal process involves blowing the gaseous mixture through one or more, preferably two, static filling columns and through one or more, preferably two, nebulization columns; preferably, the static packed and nebulization columns are arranged alternately. In a particular version of the invention, the gaseous mixture is blown through the items listed below, in the following order:

    a. bubbler
    b. static filling column
    c. nebulization column

where items b. and c. may be repeated one or more times.

Alternatively, the gaseous mixture is blown through the items listed below, in the following order:

    a. bubbler
    b. nebulization column
    c. static filling column

where items b. and c. may be repeated one or more times.

In a particularly preferred version of the invention, the gaseous mixture is blown through the items listed below, in the following order:

    a. bubbler
    b. static filling column
    c. nebulization column
    d. static filling column
    e. nebulization column
    f. static filling column

The gas outflowing from the last column (7) is essentially free from ethylene oxide; in fact, this gas has been completely converted into non-toxic substances following its reaction with the decomposing solution.

Generally, a gaseous mixture of ethylene oxide in an inert gas, such as $CO_2$, is used to decontaminate the materials and the atmosphere after the ethylene oxide sterilization is completed. The solution capable to decompose ethylene oxide is preferably an acidic solution, especially a sulphuric acid solution (for example, 40% weight/volume), in water or other solvent capable of dissolving sulphuric acid; the acidic solution reacts with ethylene oxide to form ethylene glycol. The reaction between the acid and ethylene oxide occurs in optimal conditions when the two components are present in the columns in an equimolar ratio.

This process applies every time it is necessary to remove ethylene oxide found in gaseous mixtures following, for example, sterilization treatments. The invention comprises a process for treating materials with residual ethylene oxide comprising a decontamination phase for the sterilized material, which uses a stream of a gaseous mixture, and a phase for removing ethylene oxide found in said mixture after the decontamination phase; ethylene oxide is removed by means of the process described above.

Furthermore, the invention comprises a new apparatus specifically designed to implement the removal process described above. This apparatus comprises the following: (i) one or more bubblers, (ii) one or more static filling columns, (iii) one or more nebulization columns; items (i), (ii), and (iii) are connected to each other to form a circuit. In addition, the apparatus comprises a system for distributing, inside the circuit, a decomposing solution for converting ethylene oxide.

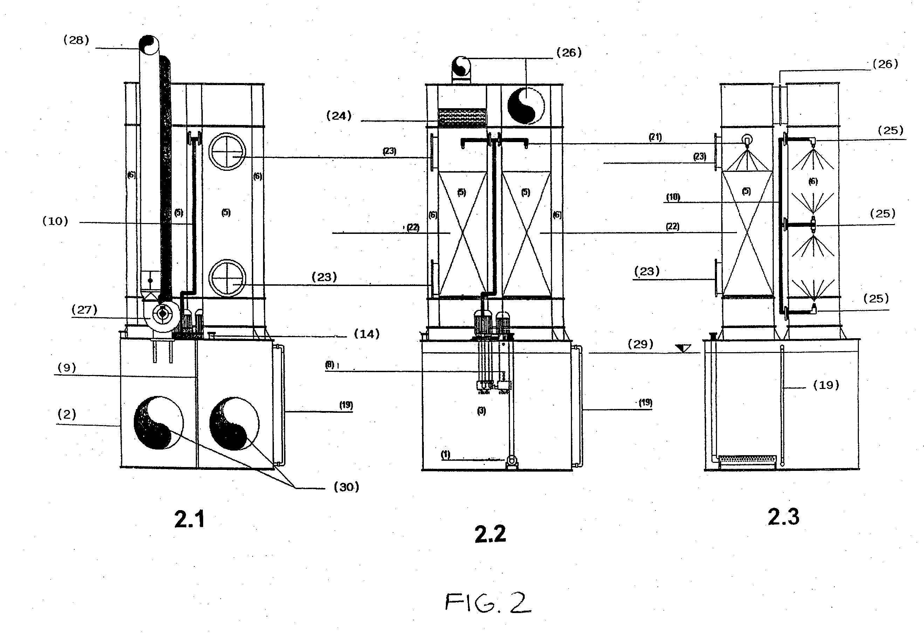

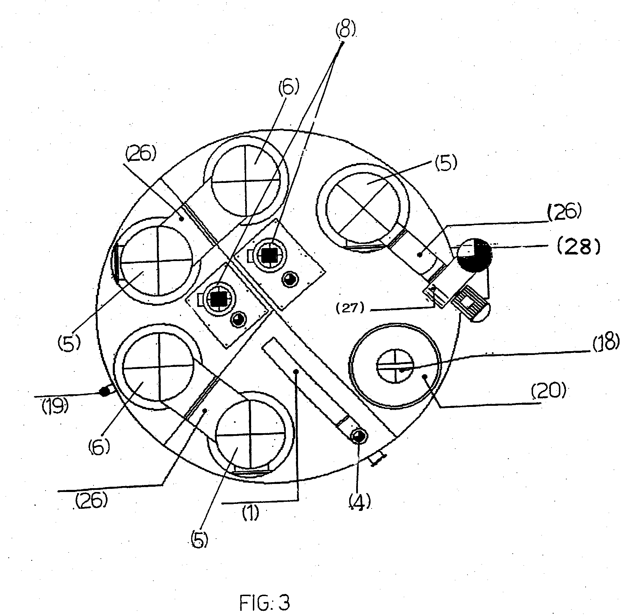

An overview of the layout and function of the parts (i)-(iii) has already been given for the process in accordance with the invention. A nonlimiting example of the apparatus in accordance with the invention is shown in figure 2 (side view) and in figure 3 (top view).

The bubbler (1) and the columns (5, 6) are attached to the top base of the tank (2). Circulation pumps (8) are also fitted onto the base of the tank; these pumps suction the contacting solution and deliver it to the columns. The tank (2) is divided into two or more compartments by separators (9); each compartment is used to supply one column. The separators can contain holes (30) for letting the decomposing solution flow from one compartment to the next: this ensures a uniform concentration of the solution contained in the tank and prevents the early unavailability of the contacting solution in the first columns of the circuit. The figures also show the level (29) of the decomposing solution.

The circulation pumps (8) draw the solution from the different sections of the tank and are connected to a piping system (10) for distributing the solution; in this way, the solution is delivered to each column as specified above.

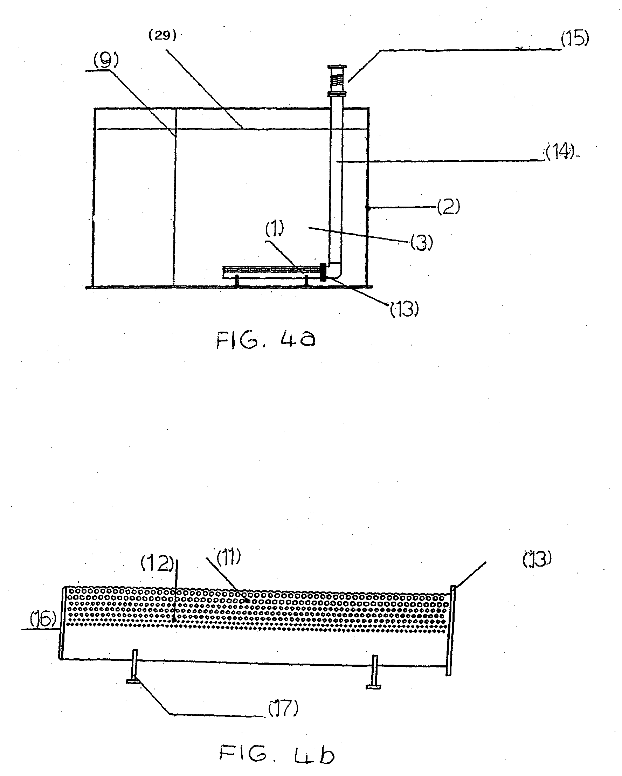

The bubbler (1) is made of inert material able to withstand the decomposing solution; for example, this material can be polypropylene and/or AISI stainless steel. A particular version of the bubbler is shown in figures 4a and 4b (detail view): the scrubber is immersed in the horizontal position, and the holes have different diameters, normally ranging between 1 and 5 mm. Specifically, the large diameter (3-5 mm on average) holes (11) are found in the top surface of the scrubber, the small diameter (1-3 mm on average) holes (12) are found in the side surface, and no holes are found in the bottom surface. One end of the bubbler is connected, by means of a flange (13), to the piping (14) that conveys the inflowing gaseous mixture; this piping may include expansion joints (15) to prevent water hammers caused by any discontinuity in the supply of gas. The other end of the scrubber is closed by a bottom (16) so that the gaseous mixture can only flow out of the microperforations. In order to increase stability, the scrubber may be anchored to the base of the tank by fastening devices (17).

The tank (see figures 2 and 3) is also equipped with gate valves for letting the solution (18) in and out of the tank, a level indicator (19), and an inspection opening (20) with seals, stainless steel bolts, and cover. Preferably, the static filling columns (5) are made of polypropylene and/or AISI stainless steel with seals, stainless steel bolts, manifolds for letting the gaseous mixture flow in and out, inspection openings, and cover. The columns are equipped with nozzles (21) (for example, made of polypropylene) at the top to supply the liquid. In addition, the columns include filling bodies (22) down which the solution flows; the shape of the filling bodies is not a determining factor; any filling body that allows a wetting liquid to flow downward is suitable for the invention. If filling bodies are not present, the solution can be made to run down the inside surface of the columns. In addition, the columns feature appropriate openings for inserting/removing (23) the filling bodies and any drops eliminators (24). The bottom base of the columns is open and communicates directly with the tank; in this way, the decomposing solution runs down the column and flows directly into the tank. Therefore, during operation, the contacting solution (3) is drawn from the tank (2) by means of the circulation pumps (8), conveyed to the top of the column (5), and delivered so that it flows down the column; then, the solution flows back into the tank from the base of the column. The process is repeated, creating a continuous cycle.

Preferably, the nebulization columns (6) are made of polypropylene and/or AISI stainless steel with seals, stainless steel bolts, manifolds for letting the gaseous mixture flow in and out, nozzles (25) positioned on different levels for supplying the solution, inspection openings with a cover. Finally, the bottom base of the columns is open and communicates directly with the tank.

Preferably, in the apparatus in accordance with the invention, the static filling and nebulization columns are arranged alternately. The preferred order of the columns in order to optimize the process was mentioned above when describing the process.

The different columns are connected to each other by means of branch pipes (26) to form a closed circuit. The last column of the series is equipped with a device (24) that prevents the decomposing solution from being dispersed in the atmosphere; this device may be, for example, a demister.

The last column is connected with the outside environment by a vertical pipe (chimney) (28) from the top of which the decontaminated gaseous mixture is released. Normally, the minimum height of the chimney is 6 m from ground level. The chimney is fitted with a centrifugal fan (27) that suctions the gas from the circuit and blows it towards the outlet. The fan only helps the gaseous mixture flow out in the final phase of the process, while the movement of the gaseous mixture inside the circuit is determined by the action of the pumps fitted in the sterilization autoclave, and/or by other pumps fitted in the circuit.

The transit speed of the gaseous mixture inside the circuit can be adjusted to suit the flow rate of the plant and the degree of contamination of the gas; generally, the speed of the gas in the circuit should not exceed 0.8 m/sec. Normally, the transit speed is chosen so that the gaseous mixture_remains at least 30 seconds in contact with the decomposing solution (i.e. remains at least 30 seconds in the section of the circuit that goes from the bubbler to the outlet of the last column).

Preferably, the piping system (10) for distributing the contacting solution is made of polypropylene and/or AINSI stainless steel. The pipes draw from the tank (2) and reach the different columns of the circuit. Then, the solution flows back into the tank from the base of the columns. The solution flows through the piping system with the aid of circulation pumps (8). Normally, the circulation pumps (8) are centrifugal pumps consisting of a body, impeller, shaft liner, outside casing, polypropylene delivery pipe, and a self-ventilating, enclosed electric motor (for example, 380 Volts, 50 Hz, ADPE, two-pole, explosion-proof motor with installed power of 5.5 Kw).

The dimensions of the apparatus are determined by the expected flow rate. In particular, to obtain optimal decontamination, the inside surface of the columns is determined by the following equation:

$$S= \frac{Q}{3600 \ (\text{sec/hr}) \ \text{x} \ V,}$$

where S = surface (m$^2$), Q = inlet flow rate of the gas (m$^3$/sec), V = transit speed of the gas (m/sec).

The apparatus described in this document was used to remove ethylene oxide contained in gaseous mixtures coming from autoclaves used to sterilize materials; it completely eliminated ethylene oxide.

**Claims**

1. A process for removing ethylene oxide contained in a gaseous mixture, **characterised in that** said gaseous mixture is blown through one or more bubblers, one or more static filling columns, and one or more nebulization columns and where, inside said bubblers and columns, the gaseous mixture comes into contact with a solution able to convert ethylene oxide into non-toxic compounds.

2. A process as claimed in claim 1, where the static filling columns and nebulization columns are arranged alternately.

3. A process as claimed in claims 1-2, where the gaseous mixture is blown through the items listed below, in the following order:

   a. bubbler
   b. static filling column
   c. nebulization column

   where items b. and c. may be repeated one or more times.

4. A process as claimed in claims 1-2, where the gaseous mixture is blown through the items listed below, in the following order:

   a. bubbler
   b. nebulization column
   c. static filling column,

   where items b. and c. may be repeated one or more times.

5. A process as claimed in claims 1-2 where the gaseous mixture is blown through the items listed below, in the following order:

   a. bubbler
   b. static filling column
   c. nebulization column
   d. static filling column
   e. nebulization column
   f. static filling column

6. A process as claimed in claims 1-5 where said static filling columns contain filling bodies that are wetted by said solution, which flows in a countercurrent direction to the gas stream.

7. A process as claimed in claims 1-6, where the last column of the series is equipped with a device that prevents the solution from being dispersed in the atmosphere.

8. A process as claimed in claim 7, where said device is a demister.

9. A process as claimed in claims 1-8, where the last column communicates with the outside environment through a chimney.

10. A process as claimed in claims 1-9, where said solution is an acidic solution.

11. A process as claimed in claim 10, where said solution is a 40% sulphuric acid solution (by weight) diluted in water or in another solvent capable of dissolving sulphuric acid.

12. An apparatus suitable for removing ethylene oxide contained in gaseous mixtures comprising: (i) one or more bubblers, (ii) one or more static filling columns, (iii) one or more nebulization columns, where items (i), (ii), and (iii) are connected to each other to form a circuit, and comprising, in addition, a system for distributing a through the circuit a solution able to decompose ethylene oxide.

13. An apparatus as claimed in claim 12, where the static filling columns and nebulization columns, are arranged alternately.

14. An apparatus as claimed in claims 12-13 comprising the items listed below, in the following order:

   a. bubbler
   b. static filling column
   c. nebulization column

   where items b. and c. may be repeated one or more times.

15. An apparatus as claimed in claims 12-13 where the gaseous mixture is blown through the items listed below, in the following order:

   a. bubbler
   b. nebulization column
   c. static filling column

   where items b. and c. may be repeated one or more times.

**16.** An apparatus as claimed in claims 12-13 where the gaseous mixture is blown through the items listed below, in the following order:

    a. bubbler
    b. static filling column
    c. nebulization column
    d. static filling column
    e. nebulization column

**17.** An apparatus as claimed in claims 12-16, where said bubbler is a microperforated pipe through which the gaseous mixture flows, the bubbler being immersed horizontally in the solution, having larger holes on its top surface, smaller holes on its side surface, and no holes on its bottom surface.

**18.** An apparatus as claimed in claims 12-17, where said static filling columns contain static filling bodies wetted by said solution, which flows in a countercurrent direction to the gas stream.

**19.** An apparatus as claimed in claims 12-18, where the last column of the series is equipped with a device that prevents the solution from being dispersed in the atmosphere.

**20.** An apparatus as claimed in claim 19, where said device is a demister.

**21.** An apparatus as claimed in claims 12-20, where the last column of the circuit communicates with the outside environment through a chimney.

**22.** An apparatus as claimed in claims 12-21, where the inside surface of each column is determined by the following equation:

$$S= \frac{Q}{3600 \ (sec/hr) \ x \ V,}$$

where S = surface ($m^2$), Q = inlet flow rate of the gas ($m^3$/sec), V = transit speed of the gas (m/sec).

**23.** A process for treating materials with ethylene oxide comprising a decontamination phase of the sterilized material by means of a gaseous mixture stream, and a phase for removing ethylene oxide found in said mixture after the decontamination phase, wherein ethylene oxide is removed by means of the process described in claims 1-11.

FIG 1

FIG. 2

EP 1 426 096 A1

FIG: 3

FIG. 4a

FIG. 4b

**European Patent Office**

## EUROPEAN SEARCH REPORT

Application Number

EP 03 02 7633

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y | WO 00/10692 A (ENVIRON INGENIEURGESELLSCHAFT ; WENZLER HORST (DE)) 2 March 2000 (2000-03-02) * abstract; claims 1,8; figure * ----- | 1-23 | B01D53/78 B01D53/75 B01D53/34 B01D53/14 A61L2/20 |
| Y | DE 196 09 204 A (DRZEVITZKY BERND ; LEITZKE HARTMUT DR (DE)) 11 September 1997 (1997-09-11) * column 4, lines 15-27 * ----- | 1-23 | |
| Y | US 5 741 470 A (WENZLER HORST) 21 April 1998 (1998-04-21) * column 2, line 13 - column 4, line 50 * ----- | 1-23 | |
| Y | "Ullmanns Encyklopädie der technischen Chemie" 1972, VERLAG CHEMIE , WEINHEIM/BERGSTR. 2 , XP002273717 * pages 589-594 * ----- | 1-23 | |

TECHNICAL FIELDS SEARCHED (Int.Cl.7)

B01D
A61L

The present search report has been drawn up for all claims

| Place of search | Date of completion of the search | Examiner |
|---|---|---|
| Munich | 17 March 2004 | Persichini, C |

EPO FORM 1503 03.82 (P04C01)

**ANNEX TO THE EUROPEAN SEARCH REPORT
ON EUROPEAN PATENT APPLICATION NO.**                    EP 03 02 7633

This annex lists the patent family members relating to the patent documents cited in the above-mentioned European search report.
The members are as contained in the European Patent Office EDP file on
The European Patent Office is in no way liable for these particulars which are merely given for the purpose of information.

17-03-2004

| Patent document cited in search report | | Publication date | Patent family member(s) | | Publication date |
|---|---|---|---|---|---|
| WO 0010692 | A | 02-03-2000 | DE | 19838327 C1 | 18-05-2000 |
| | | | WO | 0010692 A1 | 02-03-2000 |
| DE 19609204 | A | 11-09-1997 | DE | 19609204 A1 | 11-09-1997 |
| | | | AU | 2689997 A | 22-09-1997 |
| | | | WO | 9732660 A2 | 12-09-1997 |
| US 5741470 | A | 21-04-1998 | DE | 4116187 A1 | 19-11-1992 |
| | | | AT | 128636 T | 15-10-1995 |
| | | | WO | 9220432 A2 | 26-11-1992 |
| | | | DE | 9218825 U1 | 02-11-1995 |
| | | | DE | 59203895 D1 | 09-11-1995 |
| | | | DK | 539551 T3 | 04-03-1996 |
| | | | EP | 0539551 A1 | 05-05-1993 |
| | | | ES | 2078742 T3 | 16-12-1995 |
| | | | GR | 3018493 T3 | 31-03-1996 |

EPO FORM P0459

For more details about this annex : see Official Journal of the European Patent Office, No. 12/82